# EUROPEAN PATENT APPLICATION

(11) **EP 1 977 730 A1**
(43) Date of publication of application: **08.10.2008**
(21) Application number: 06842872.1
(22) Date of filing: 19.12.2006
(51) Int. Cl.: A61K 8/35, A61K 8/40, A61K 8/49, A61Q 17/04

(54) **SUNSCREEN COSMETICS**

(30) Priority: 30.12.2005 JP 2005380696; 30.12.2005 JP 2005380697
(71) Applicant: SHISEIDO COMPANY, LTD., Tokyo 104-8010 (JP)
(72) Inventor: ARAKI, Hidefumi, Yokohama-shi Kanagawa 2248558 (JP); ABE, Koji, Yokohama-shi Kanagawa 2248558 (JP); YAJIMA, Isao, Yokohama-shi Kanagawa 2248558 (JP); KAKOKI, Hiroyuki, Yokohama-shi Kanagawa 2248558 (JP); OGUCHI, Nozomi, c/o Shiseido Research Center 2-1 Hayabuchi 2-chome, Tsuzuki-ku (JP)
(74) Representative: Merkle, Gebhard
(86) International application number: PCT/JP2006/325268
(87) International publication number: WO 2007/077729

(57) **Abstract**

The present invention is a sunscreen cosmetic comprising 4-(1,1-dimethylethyl)-4'-methoxydibenzoylmethane, a benzotriazole derivative represented by the following general formula (I), and ethylhexyl 2-cyano-3,3-diphenylacrylate.

The present invention is a sunscreen cosmetic comprising hexyl diethylaminohydroxybenzoylbenzoate and a benzotriazole derivative represented by the following general formula (I). 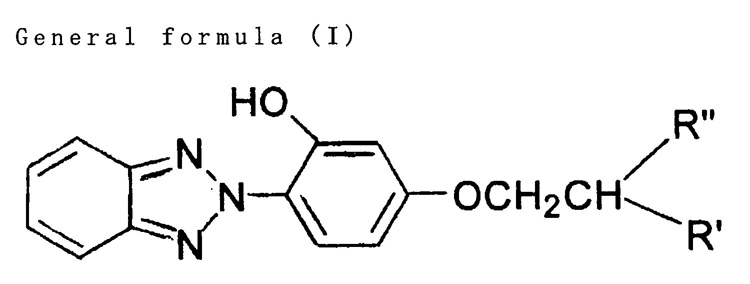 (In this formula, R' = a straight chain alkyl group of C1-C6 and R" = a straight chain alkyl group of C1-C3.)

The object of the present invention is to provide a sunscreen cosmetic that prevents staining due to secondary adhesion to clothing.

## Description

### TECHNICAL FIELD

The present invention relates to a sunscreen cosmetic. More specifically, it relates to a sunscreen cosmetic that prevents staining due to secondary adhesion to clothing.

### BACKGROUND ART

Important ultraviolet wavelength regions absorbed by sunscreen cosmetics are the UV-A region (320-400 nm) and UV-B region (290-320 nm). It was believed that the ultraviolet light in the UV-A region darkened the skin but it would not cause sunburn and accelerate aging of the skin as the ultraviolet light in the UV-B region would.

However, in recent years, it has been made clear that, whereas the ultraviolet light in the UV-B region only reaches the surface part of the skin, the ultraviolet light in the UV-A region reaches the deeper part of the skin and induces not only skin aging but also skin cancer. Therefore, there is an increasing demand for sunscreen cosmetics to have ultraviolet absorption in the UV-A region.

There are many kinds of ultraviolet absorbents that are added to sunscreen cosmetics. 4-(1,1-dimethylethyl)-4'-methoxydibenzoylmethane is used as an ultraviolet absorbent in the UV-A region. Hexyl diethylaminohydroxybenzoylbenzoate is also used.

An endermic liniment that has superior ultraviolet absorption in the UV-A region as well and prevents the coloring tendency of 2-ethylhexyl p-methoxycinnamate thus improving stability of the ultraviolet absorption effect has been developed by adding a benzotriazole derivative represented by the general formula (I) to an endermic liniment containing 2-ethylhexyl p-methoxycinnamate as an ultraviolet absorbent in the UV-B region (Patent document 1).

Patent Citation 1: Japanese Patent Laid-Open 2005-206473 bulletin

### DISCLOSURE OF INVENTION

### TECHNICAL PROBLEM

### [First invention: Claims 1-2]

4-(1, 1-dimethylethyl)-4'-methoxydibenzoylmethane is a useful ultraviolet absorbent in the UV-A region, but there is a problem in that the addition of this to a sunscreen cosmetic tends to result in staining clothing from secondary adhesion. Therefore, 4-(1,1-dimethylethyl)-4'-methoxydibenzoylmethane cannot be added to a sunscreen cosmetic at a high blend ratio.

In view of the aforementioned problem, the inventors conducted earnest research and discovered that a sunscreen cosmetic that reduces the staining tendency of 4-(1,1-dimethylethyl)-4'-methoxydibenzoylmethane on clothing and exhibits exceptional ultraviolet absorption properties in the UV-A region and UV-B region can be provided by adding 4-(1,1-dimethylethyl)-4'-methoxydibenzoylmethane, a benzotriazole derivative of a specific structure, and ethylhexyl 2-cyano-3,3-diphenylacrylate to a sunscreen cosmetic, thus completing the present invention.

The object of the present invention is to provide a sunscreen cosmetic that reduces the staining tendency of 4-(1,1-dimethylethyl)-4'-methoxydibenzoylmethane on clothing and exhibits superior ultraviolet absorption properties in the UV-A region and UV-B region.

### [Second invention: Claims 3-5]

Hexyl diethylaminohydroxybenzoylbenzoate is a useful ultraviolet absorbent in the UV-A region, but there is a problem in that the addition of this to a sunscreen cosmetic tends to result in staining clothing from secondary adhesion. Therefore, hexyl diethylaminohydroxybenzoylbenzoate cannot be added to a sunscreen cosmetic at a high blend ratio.

In view of the aforementioned problem, the inventors conducted earnest research and discovered that a sunscreen cosmetic that reduces the staining tendency of hexyl diethylaminohydroxybenzoylbenzoate on clothing can be provided by adding hexyl diethylaminohydroxybenzoylbenzoate and a benzotriazole derivative of a specific structure to a sunscreen cosmetic, thus completing the present invention.

The object of the present invention is to provide a sunscreen cosmetic that reduces the staining tendency of hexyl diethylaminohydroxybenzoylbenzoate on clothing and works well particularly in the UV-A region.

### TECHNICAL SOLUTION

### [First invention]

That is, the present invention provides a sunscreen cosmetic comprising 4-(1,1-dimethylethyl)-4'-methoxydibenzoylmethane, a benzotriazole derivative represented by the following general formula (I), and ethylhexyl 2-cyano-3,3-diphenylacrylate.

### General formula (I)

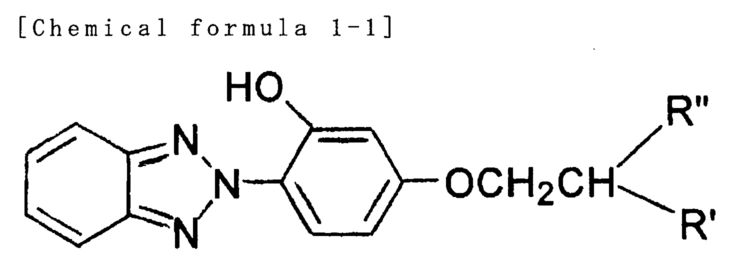

(In this formula, R' = a straight chain alkyl group of C1-C6 and R" = a straight chain alkyl group of C1-C3.)

Also, the present invention provides the aforementioned sunscreen cosmetic wherein said benzotriazole derivative is one, two or more chosen from a group consisting of 2-[4-(2-ethylhexyloxy)-2-hydroxyphenyl]-2H-benzotriazole and 2-(2-hydroxy-4-isobuthoxyphenyl)-2H-benzotriazole.

### [Second invention]

That is, the present invention provides a sunscreen cosmetic comprising hexyl diethylaminohydroxybenzoylbenzoate and a benzotriazole derivative represented by the following general formula (I).

### General formula (I)

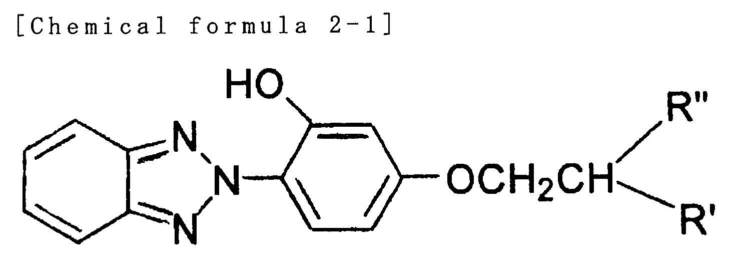

(In this formula, R' = a straight chain alkyl group of C1-C6 and R" = a straight chain alkyl group of C1-C3.)

Also, the present invention provides the aforementioned sunscreen cosmetic wherein said benzotriazole derivative is one, two or more chosen from a group consisting of 2-[4-(2-ethylhexyloxy)-2-hydroxyphenyl]-2H-benzotriazole and 2-(2-hydroxy-4-isobuthoxyphenyl)-2H-benzotriazole.

Furthermore, the present invention provides the aforementioned sunscreen cosmetic also comprising ethylhexyl 2-cyano-3,3-diphenylacrylate.

### ADVANTAGEOUS EFFECTS

### [First and second inventions]

(1) The sunscreen cosmetic of the present invention reduces the staining tendency of 4-(1,1-dimethyethyl)-4'-methoxydibenzoylmethane or hexyl diethylaminohydroxybenzoylbenzoate on clothing. Therefore, it can be added to a sunscreen cosmetic at a high blend ratio.
(2) The sunscreen cosmetic of the present invention exhibits high ultraviolet absorption capacity in a broad region from UV-A to UV-B.

### BRIEF DESCRIPTION OF DRAWINGS

FIG. 1 illustrates how to measure the staining tendency.
FIG. 2 shows the measurement results of the staining tendency of the first invention.
FIG. 3 shows the measurement results of the staining tendency of the second invention.

### BEST MODE FOR CARRYING OUT THE INVENTION

The present invention is described in detail below.

### [First invention]

### "4-(1,1-dimethylethyl)-4'-methoxydibenzoylmethane"

The dibenzoylmethane derivative used in the present invention is 4-(1,1-dimethylethyl)-4'-methoxydibenzoylmethane. In the present invention, a commercial product (Parsol 1789 from DSM Nutritional Products) can be used.

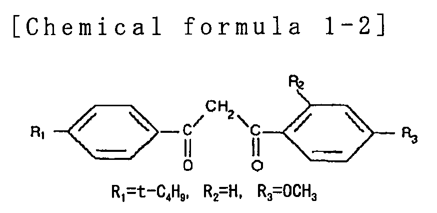

### "Benzotriazole derivative represented by general formula (I)"

The benzotriazole derivative of general formula (I) is a prior art chemical compound, which is synthesized in the following manner. A common method is to use sodium nitrite and such to turn o-nitroaniline into a diazonium salt and then couple this with phenol to synthesize a monoazo compound, and reduce it to obtain benzotriazole.

### (Method A)

### First process

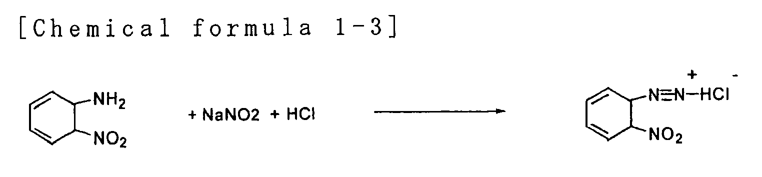

### Second process

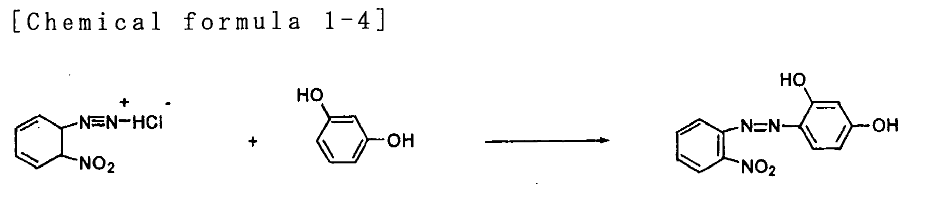

### Third process

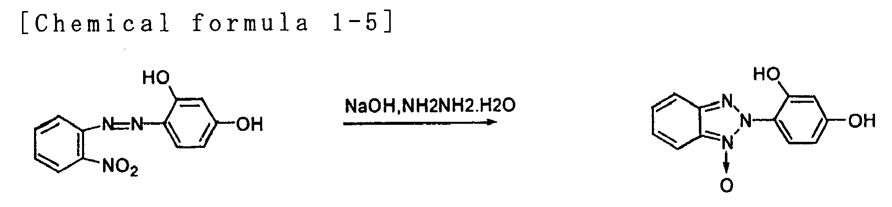

### Fourth process

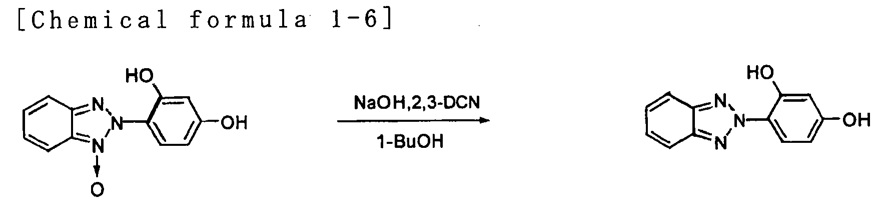

(In this formula, 2,3-DCN denotes 2,3-dichloro-1,4-naphthoquinone.)

### Fifth process

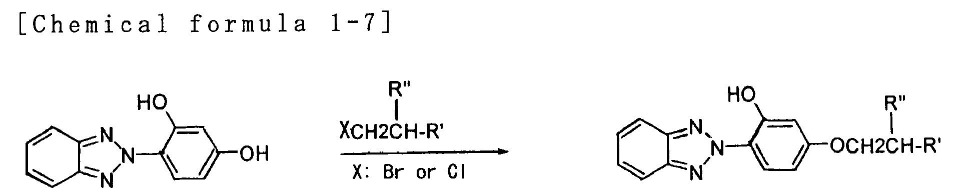

(In this formula, R = H or CH3, R' = a straight chain alkyl group of C1-C6 and R" = a straight chain alkyl group of C1-C3.)

### (Method B)

### First process

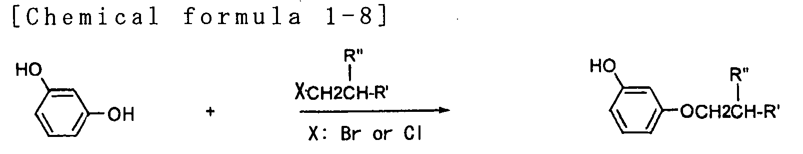

### Second process

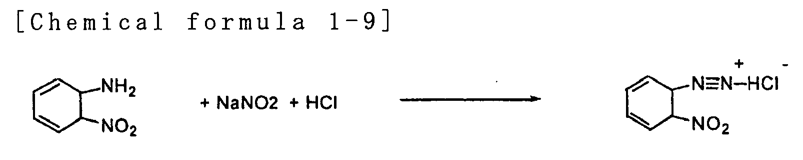

### Third process

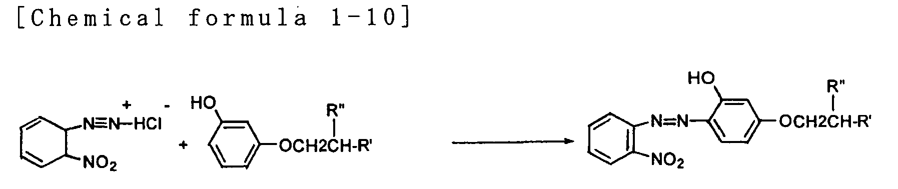

### Fourth process

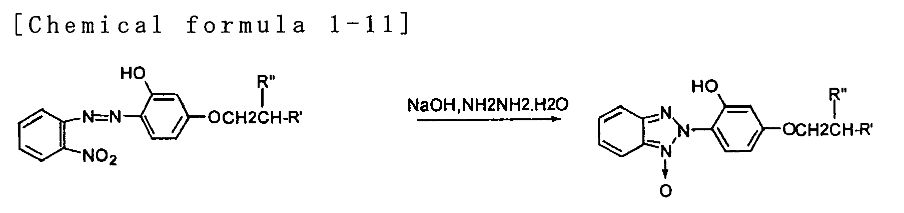

### Fifth process

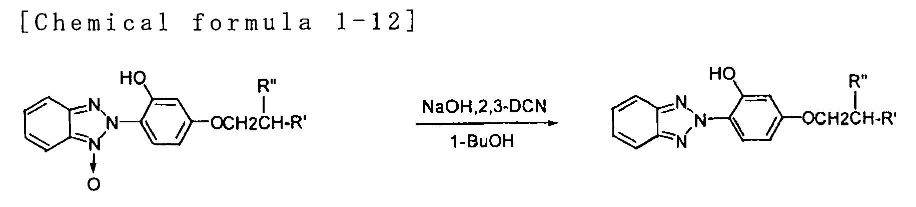

(In this formula, R' = a straight chain alkyl group of C1-C6 and R" = a straight chain alkyl group of C1-C3. 2,3-DCN denotes 2,3-dichloro-1,4-naphthoquinone.)

Refluxing a corresponding benzotriazole and alkylated halogen in a mixed solvent of methylisobutylketone and dimethylformamide produces the compound of general formula (I) with a particularly high yield.
Specifically, 6-(2H-bonzotriazole-2-yl) resorcinol is put into a four-neck flask equipped with a thermometer and refluxing cooler, to which methylisobutylketone and dimethylformamide are added and the mixture is stirred. To this, sodium carbonate and 2-ethylhexyl bromide are added and the temperature is raised to the refluxing temperature while stirring. After the mixture is stirred while the refluxing temperature is maintained for a prescribed amount of time, methylisobutylketone is recovered under normal pressure; the remaining oil is then rinsed with water to remove excess sodium carbonate and inorganic byproducts to obtain liquid 2-[4-(2-ethylhexyloxy)-2-hydroxyphenyl]-2H-benzotriazole at a high yield.

### "Ethylhexyl 2-cyano-3,3-diphenylacrylate"

Ethylhexyl 2-cyano-3,3-diphenylacrylate is a prior art ultraviolet absorbent represented by the following formula. In the present invention, a commercial product (Octocrylene from DSM Nutrition Co., Ltd.) can be used.

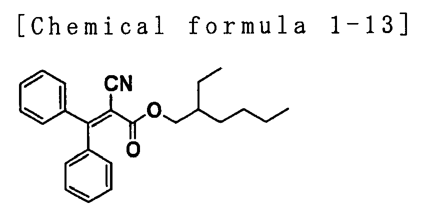

The blend ratio of 4-(1,1-dimethylethyl)-4'-methoxydibenzoylmethane is chosen as appropriate for the target product; it is preferably 0.5-5 wt%, more preferably 1-3 wt%, of the total amount of the sunscreen cosmetic. The significance of the present invention is particularly great when the blend ratio is high, i. e. 2 wt% or more.

The blend ratio of the benzotriazole derivative represented by general formula (I) is chosen as appropriate for the target product; it is preferably 0.5-10 wt%, more preferably 1-5 wt%, of the total amount of the sunscreen cosmetic.

The blend ratio of ethylhexyl 2-cyano-3,3-diphenylacrylate is chosen as appropriate for the target product; it is preferably 0.5-10 wt%, more preferably 1-5 wt%, of the total amount of the sunscreen cosmetic.

### [Second invention]

### "Hexyl diethylaminohydroxybenzoylbenzoate"

Hexyl diethylaminohydroxybenzoylbenzoate used in the present invention is an ultraviolet absorbent in the UV-A region represented by the following formula. In the present invention, a commercial product (Uvinul A plus from BASF) can be used.

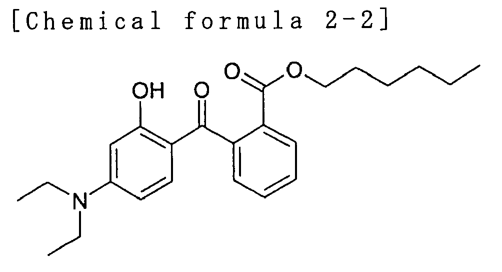

### "Benzotriazole derivative represented by general formula (I)"

The benzotriazole represented by general formula (I) is the same as described in [First invention].

### "Ethylhexyl 2-cyano-3,3-diphenylacrylate"

Ethylhexyl 2-cyano-3,3-diphenylacrylate is a prior art ultraviolet absorbent in the UV-B region represented by the following formula. By additionally blending in this ultraviolet absorbent, a sunscreen cosmetic that has an exceptional ultraviolet absorption capacity in the UV-A region as well as high ultraviolet absorption capacity in a broad region including the UV-B region can be provided. In the present invention, a commercial product (Octocrylene from DSM Nutrition Co., Ltd.) can be used.

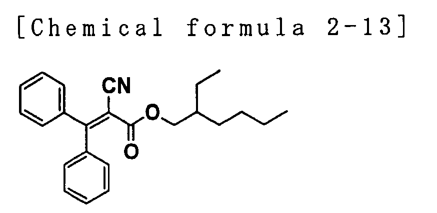

The blend ratio of hexyl diethylaminohydroxybenzoylbenzoate is chosen as appropriate for the target product; it is preferably 0.5-5 wt%, more preferably 1-3 wt%, of the total amount of the sunscreen cosmetic. The significance of the present invention is particularly great when the blend ratio is high, i. e. 2 wt% or more.

The blend ratio of the benzotriazole derivative represented by general formula (I) is chosen as appropriate for the target product; it is preferably 0.5-10 wt%, more preferably 1-5 wt%, of the total amount of the sunscreen cosmetic.

The blend ratio of ethylhexyl 2-cyano-3,3-diphenylacrylate is chosen as appropriate for the target product; it is preferably 0.5-10 wt%, more preferably 1-5 wt%, of the total amount of the sunscreen cosmetic.

### [First and second inventions]

In addition to the aforementioned essential ingredients, other ingredients commonly used in cosmetics can be blended in as necessary in the sunscreen cosmetic of the present invention; examples of such ingredients include whitening agents, humectants, antioxidants, oil-based ingredients, other ultraviolet absorbents, surfactants, thickeners, alcohols, powder ingredients, coloring agents, water-based ingredients, water, and various skin nutrients, and the sunscreen cosmetic can be prepared with a conventional method. Specific examples include the following ingredients:

Oil components such as avocado oil, macadamia nut oil, corn oil, olive oil, rapeseed oil, evening primrose oil, castor oil, sunflower oil, tea seed oil, rice bran oil, jojoba oil, cacao oil, coconut oil, squalene, beef tallow, Japanese core wax, beeswax, candelilla wax, carnauba wax, whale wax, lanolin, liquid paraffin, polyoxyethylene (8 mole) oleyl alcohol ether, glyceryl monooleate, cyclomethicone, dimethylpolysiloxane, and diphenylpolysiloxane.
Higher alcohols such as caprylic alcohol, lauryl alcohol, myristyl alcohol, cetyl alcohol, cholesterol, and phytosterol.
Higher fatty acids such as caprylic acid, lauric acid, myristic acid, palmitic acid, stearic acid, behenic acid, lanolin fatty acid, linoleic acid, and linolenic acid.
Humectants such as polyethylene glycol, glycerin, sorbitol, xylitol, maltitol, mucopolysaccharide, hyaluronic acid, chondroitin sulfate, and chitosan.
Thickeners such as methyl cellulose, ethyl cellulose, Arabic gum, and polyvinyl alcohol.
Organic solvents such as ethanol and 1,3-butylene glycol.
Antioxidants such as butylhydroxytoluene, tocopherol, and phytic acid.
Antibacterial preservatives such as benzoic acid, salicylic acid, sorbic acid, paraoxybenzoic esters (ethylparaben and butylparaben, for example), and hexachlorophene.
Amino acids such as glycine, alanine, valine, leucine, serine, threonine, phenyalanine, tyrosine, aspartic acid, asparagine, glutamine, taurine, arginine, and histidine, as well as hydrochlorides thereof.
Organic acids such as acyl sarcosinic acid (sodium lauroyl sarcosinate, for example), glutathione, citric acid, malic acid, tartaric acid, and lactic acid.
Vitamins such as vitamin A and its derivatives, vitamin B's including vitamin B6 hydrochloride, vitamin B6 tripalmitate, vitamin B6 dioctanoate, vitamin B2 and its derivatives, vitamin B12, and vitamin B15 and its derivatives, vitamin C's including ascorbic acid, ascorbic malic esters (salts), and ascorbic dipalmitate, vitamin E's including α-tocopherol, β-tocopherol, γ-tocopherol, vitamin E acetate, and vitamin E nicotinate, vitamin D's, vitamin H, pantothenic acid, and pantethine.
Various drugs such as nicotinamide, benzyl nicotinate, γ-oryzanol, allantoin, glycyrrhizic acid (salt), glycyrrhizic acid and its derivatives, hinokitiol, musidine, bisabolol, eucalyptol, thymol, inositol, saponins (saikosaponin, carrot saponin, gourd saponin, soapberry saponin, etc.) pantothenylethyl ether, ethynylestradiol, tranexamic acid, cepharanthine, and placenta extract.
Natural extracts from Rumex japonicus, Sophora flavescens, Nuphar japonica, orange, sage, thyme, yarrow, mallow, smilax, swertia, Ligusticum acutilobum, bitter orange peel, birch, horsetail, gourd, horse chestnut, creeping saxifrage, arnica, lily, mugwort, Paeonia lactiflora, aloe, gardenia, Chamaecyparis pisifera, etc. extracted by using an organic solvent, alcohol, polyhydric alcohol, water, hydroalcohol, etc.
Cation surfactants such as stearyltrimethylammonium chloride, benzalkonium chloride, and lauryl amine oxide.
Sequestering agents such as disodium edetate, trisodium edetate, sodium citrate, sodium polyphosphate, sodium metaphosphate, and gluconic acid.
Perfumes, scrubbing agents, purified water, etc.

Particularly preferable base agents for the sunscreen cosmetic of the present invention are oil components including decamethylcyclopentasiloxane, isononyl isononanoate, dimethylpolysiloxane, heptamethyloctyltrisiloxane, trimethylsiloxysilicic acid, liquid paraffin, squalane, cetyl isooctanoate, triglyceride octanoate, and di-2-ethylhexyl succinate. The present invention is used preferably in sunscreen cosmetics that use decamethylcyclopentasiloxane as the main base agent.

The sunscreen cosmetic of the present invention can be used in any product form such as ointments, cream, emulsion, and lotions. The dosage form is not limited either.

### EXAMPLES

The invention is described in specific detail through Examples. The present invention is not limited to these Examples.

### [First invention]

### <Synthesis example of the benzotriazole derivative represented by general formula (I)>

### "Synthesis example 1-1: Synthesis of 2-[4-(2-ethylhexyloxy)-2-hydroxyphenyl]-2H-benzotriazole"

45.4 g (0.20 moles) of 6-(2H-benzotriazole-2-yl) resorcinol, synthesized by using a conventional method, was put into a 500-ml four neck flask equipped with a thermometer and a reflux cooling apparatus, to which 50 ml of methylisobutylketone and 4.0 g of dimethylformamide were added, followed by stirring. Into this, 25.4 g (0.24 moles) of sodium carbonate and 77.2 g (0.40 moles) of 2-ethylhexyl bromide were added and the mixture was heated up to the refluxing temperature while being stirred. After stirring the mixture for 15 hours while maintaining the refluxing temperature, methylisobutylketone was recovered under normal pressure and the remaining oil was then rinsed with water to remove excess sodium carbonate and inorganic byproducts. This oil was distilled under a reduced pressure to obtain 52.1 g of a 220-225 ° C/0.2-0.3 mmHg fraction, which was yellow and transparent. This compound was liquid at ordinary temperatures; the yield was 76.7% and the HPLC purity was 99.0%.

### "Synthesis example 1-2: Synthesis of 2-[2-hydroxy-4-isobutoxyphenyl]-2H-benzotriazole"

Instead of 2-ethylhexyl bromide, the equal number of moles of isobutyl bromide was used in the same manner as in Synthesis example 1. Slightly yellow-gray-white powdery crystals were obtained at a yield of 72.5%. The m.p. was 120.0-120.8 ° C, λ max = 345.6 nm, and ε = 21750.

W/0 sunscreens shown in Table 1-1 were prepared using a conventional method and the staining tendency due to secondary adhesion was investigated.

**[Table 1-1]**

| | Comparative example | | Example | | |
|---|---|---|---|---|---|
| | 1-1 | 1-2 | 1-1 | 1-2 | 1-3 |
| Decamethylcyclopentasiloxane | 26. 0 | 21.0 | 20.0 | 18.0 | 16.0 |
| Dimethylpolysiloxane | 2.0 | 2.0 | 2.0 | 2.0 | 2.0 |
| Lauryl PEG-9 polydimethylsiloxyethyl dimethicone | 1.5 | 1.5 | 1.5 | 1.5 | 1.5 |
| Trimethylsiloxysilicic acid | 5.0 | 5.0 | 5.0 | 5.0 | 5.0 |
| Polyoxyethylene/ methylpolysiloxane copolymer | 0.5 | 0.5 | 0.5 | 0.5 | 0.5 |
| Triglyceride isooctanoate. | 5.0 | 5.0 | 5.0 | 5.0 | 5.0 |
| Cetyl isooctanoate | 5. 0 | 5. 0 | 5. 0 | 5. 0 | 5.0 |
| Isononyl isononanoate | 5.0 | 5.0 | 5.0 | 5.0 | 5.0 |
| 2-ethylhexyl paramethoxycinnamate | 7.5 | 7.5 | 7.5 | 7.5 | 7.5 |
| Octocrylene | - | 5. 0 | 5. 0 | 5.0 | 5.0 |
| 4-(1,1-dimethylethyl)-4'-methoxydibenzoylmethane | 2.0 | 2.0 | 2.0 | 2.0 | 2.0 |
| Benzotriazole derivative *1 | - | - | 1.0 | 3.0 | 5. 0 |
| Dimethyldistearyl ammonium hectorite | 0.2 | 0.2 | 0.2 | 0.2 | 0.2 |
| Hydrophobicized titanium oxide | 12.0 | 12.0 | 12.0 | 12.0 | 12.0 |
| Purified water | 18.7 | 18.7 | 18. 7 | 18.7 | 18.7 |
| Trisodium edetate | 0.05 | 0.05 | 0.05 | 0.05 | 0.05 |
| Dipropylene glycol | 5.0 | 5.0 | 5. 0 | 5.0 | 5.0 |
| Paraben | 0. 25 | 0.25 | 0.25 | 0.25 | 0.25 |
| Glycerin | 2.0 | 2.0 | 2.0 | 2.0 | 2.0 |
| Ethanol | 2.0 | 2.0 | 2. 0 | 2.0 | 2.0 |
| Phenoxyethanol | 0.3 | 0.3 | 0.3 | 0.3 | 0.3 |
| Total | 100.0 | 100.0 | 100.0 | 100.0 | 100.0 |

| | | | | | |
|---|---|---|---|---|---|
| *1: 2-[4-(2-ethylhexyloxy)-2-hydroxyphenyl]-2H-benzotriazole | | | | | |

### "Method for measuring the staining tendency"

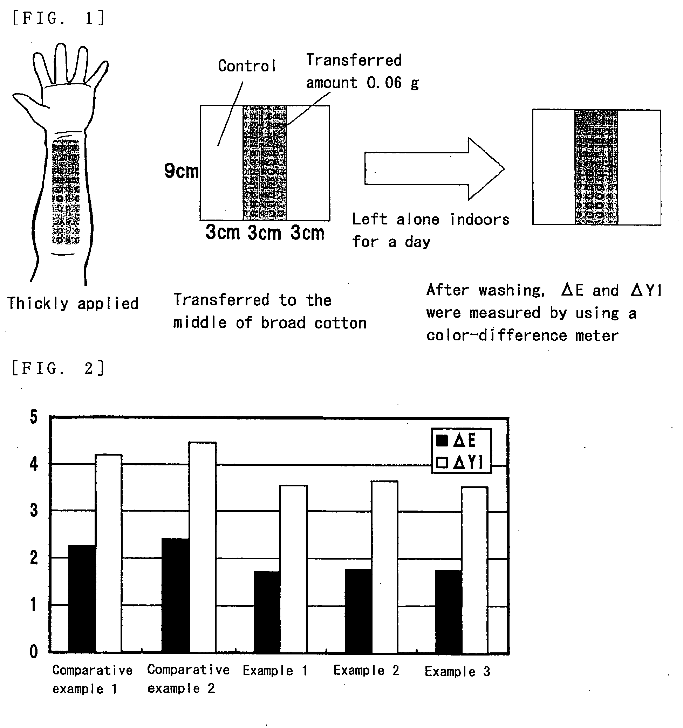

As shown in FIG. 1, the sample was applied thickly on an arm and then transferred to the middle of broad cotton (transferred amount approximately 0.06 g); after being left alone indoors for a day, it was washed using a conventional laundry detergent and Δ E and Δ YI were measured by using a spectrophotometer (CM-2002 from Minolta, currently Konica Minolta Sensing Co., Ltd.). The results are shown in Figure 2.

FIG. 2 indicates that the staining tendency of 4-(1,1-dimethylethyl)-4'-methoxydibenzoylmethane decreases by the addition of the benzotriazole derivative of General formula (I). Even when the blend ratio is high, the staining tendency decreases compared with the case in which it is not added.

The following are formulation examples of the sunscreen cosmetic of the present invention. Each of these examples is a sunscreen cosmetic that has a reduced staining tendency on clothing and exhibits superior ultraviolet absorption capacity in a broad region from UV-A to UV-B.

### "Example 1-4: Sunscreen cosmetic W/O emulsion"

| | | wt% |
|---|---|---|
| 1. | Dimethylpolysiloxane | 1 |
| 2. | Decamethylcyclopentasiloxane | 25 |
| 3. | Trimethylsiloxysilicic acid | 5 |
| 4. | Polyoxyethylene-methylpolysiloxane copolymer | 1 |
| 5. | Lauryl PEG-9 polydimethylsiloxyethyl dimethicone | 1 |
| 6. | Isononyl isononoate | 5 |
| 7. | Dipropylene glycol | 5 |
| 8. | Dipotassium glycyrrhizate | 0.02 |
| 9. | Glutathione | 1 |
| 10. | Thiotaurine | 0.05 |
| 11. | Sophora flavescens extract | 1 |
| 12. | Paraben | Appropriate amount |
| 13. | Phenoxyethanol | Appropriate amount |
| 14. | 2-ethylhexyl paramethoxycinnamate | 7.5 |
| 15. | Dimethyldistearylammonium hectorite | 0.5 |
| 16. | Spherical polyalkyl acrylate powder | 5 |
| 17. | Butylethylpropanediol | 0.5 |
| 18. | 4-(1,1-dimethylethyl)-4'-methoxydibenzoylmethane | 2 |
| 19. | Ethylhexyl 2-cyano-3,3-diphenylacrylate | 5 |
| 20. | Benzotriazole derivative of Synthesis example 2 | 5 |
| 21. | Hydrophobicized zinc oxide | 15 |
| 22. | Purified water | Balance |

### Preparation method

The water phase was gradually added to the oil phase; after the addition a stirrer was used to homogenize the emulsified particles to complete the preparation.

### "Example 1-5: Sunscreen cosmetic 0/W emulsion"

| | | wt% |
|---|---|---|
| 1. | Polyoxyethylene hydrogenated castor oil | 1 |
| 2. | Dimethicone copolyol | 0.5 |
| 3. | Decamethylcyclopentasiloxane | 15 |
| 4. | Isostearic acid | 0.5 |
| 5. | Phenyl trimethicone | 1 |
| 6. | Hydrophobicized titanium oxide | 5 |
| 7. | 4-(1,1-dimethylethyl)-4'-methoxydibenzoylmethane | 3 |
| 8. | Ethylhexyl 2-cyano-3,3-diphenylacrylate | 5 |
| 9. | 2-ethylhexyl-paramethoxycinnamate | 5 |
| 10. | Benzotriazole derivative of Synthesis example 1 | 2 |
| 11. | Silica | 1 |
| 12. | Citric acid | 0.01 |
| 13. | Sodium citrate | 0.09 |
| 14. | Paraben | Appropriate amount |
| 15. | Phenoxyethanol | Appropriate amount |
| 16. | Ethanol | 5 |
| 17. | Dynamite glycerin | 1 |
| 18. | Succinoglucan | 0.2 |
| 19. | Cellulose gum | 1 |
| 20. | Ion-exchanged water | Balance |

### Preparation method

The water phase, 11-20, was prepared, which was then gradually added to the oil phase, 1-10, followed by stirring by means of a homomixer.

### "Example 1-6: Sunscreen cosmetic O/W/O emulsion"

| | | wt% |
|---|---|---|
| 1. | Dimethylethyl polyoxyethylene (50) polyoxypropylene (40) block copolymer | 0.5 |
| 2. | Isostearic acid | 0.2 |
| 3. | 2-ethylhexyl paramethoxycinnamate | 7.5 |
| 4. | Benzotriazole derivative of Synthesis example 2 | 2.0 |
| 5. | 4-(1,1-dimethylethyl)-4'- methoxydibenzoylmethane | 2.0 |
| 6. | Ethylhexyl 2-cyano-3,3-diphenylacrylate | 5.0 |
| 7. | Decamethylcyclopentasiloxane | 35 |
| 8. | Polyoxybutylene polyoxypropylene glycol | 2.0 |
| 9. | Dimethyldistearylammonium hectorite | 0.5 |
| 10. | Hydrophobicized titanium oxide | 12.0 |
| 11. | Citric acid | 0.04 |
| 12. | Sodium citrate | 0.06 |
| 13. | Dipropylene glycol | 2.0 |
| 14. | Methyl glucose | 1.0 |
| 15. | Dynamite glycerin | 1.0 |
| 16. | Sodium chloride | 0.1 |
| 17. | Methylparaben | Appropriate amount |
| 18. | Phenoxyethanol | Appropriate amount |
| 19. | Ion-exchanged water | Balance |

### Preparation method

The oil phase, 1-6, was gradually added to the water phase, 11-19, to obtain an 0/W preparation. This preparation was gradually added to the oil phase consisting of 7-10, followed by stirring using a homomixer to obtain the target emulsion.

### "Example 1-7: Self tanning cosmetic"

| | | wt% |
|---|---|---|
| Part A | | |
| 1. | 1,3-butylene glycol | 5 |
| 2. | Glycerin | 2 |
| 3. | Dihydroxyacetone | 5 |
| 4. | Disodium edetate | 0.05 |
| 5. | Paraben Appropriate | amount |
| 6. | Ion-exchanged water | Balance |

| Part B | | |
|---|---|---|
| 7. | Glyceryl stearate | 4 |
| 8. | Behenyl alcohol | 3 |
| 9. | Stearyl alcohol | 2 |
| 10. | Silicone oil | 1 |
| 11. | Hydrogenated palm oil | 2 |
| 12. | Liquid paraffin | 7 |
| 13. | Benzotriazole derivative of Synthesis example 2 | 2 |
| 14. methoxydibenzoylmethane | 4-(1,1-dimethylethyl)-4'- | 2 |
| 15. | Ethylhexyl 2-cyano-3,3-diphenylacrylate 5 | 5 |
| 16. | Perfume | Appropriate amount |

### Preparation method

Disodium edetate, dihydroacetone, glycerin, and paraben heated and dissolved in 1,3-butylene glycol were added to the ion-exchanged water of part A. Each ingredient of part B was thoroughly dissolved and heated, and then added to part A, followed by emulsification. This was cooled to obtain the self tanning cream.

### [Second invention]

### <Synthesis example of the benzotriazole derivative represented by general formula (I)>

### "Synthesis example 2-1: Synthesis of 2-[4-(2-ethylhexyloxy)-2-hydroxyphenyl]-2H-benzotriazole"

45.4 g (0.20 moles) of 6-(2H-benzotriazole-2-yl) resorcinol, synthesized by using a conventional method, was put into a 500-ml four neck flask equipped with a thermometer and a reflux cooling apparatus, to which 50 ml of methylisobutylketone and 4.0 g of dimethylformamide were added, followed by stirring. Into this, 25.4 g (0.24 moles) of sodium carbonate and 77.2 g (0.40 moles) of 2-ethylhexyl bromide were added and the mixture was heated up to the refluxing temperature while being stirred. After stirring the mixture for 15 hours while maintaining the refluxing temperature, methylisobutylketone was recovered under normal pressure and the remaining oil was then rinsed with water to remove excess sodium carbonate and inorganic byproducts. This oil was distilled under a reduced pressure to obtain 52.1 g of a 220-225 ° C/0.2-0.3 mmHg fraction, which was yellow and transparent. This compound was liquid at ordinary temperatures; the yield was 76.7% and the HPLC purity was 99.0%.

### "Synthesis example 2-2: Synthesis of 2-[2-hydroxy-4-isobutoxyphenyl]-2H-benzotriazole"

Instead of 2-ethylhexyl bromide, the equal number of moles of isobutyl bromide was used in the same manner as in Synthesis example 1. Slightly yellow-gray-white powdery crystals were obtained at a yield of 72.5%. The m.p. was 120.0-120.8 ° C, λ max = 345.6 nm, and ε = 21750.

W/0 sunscreens shown in Table 2-1 were prepared using a conventional method and the staining tendency due to secondary adhesion was investigated.

**[Table 2-1]**

| | Comparative example | Example | | |
|---|---|---|---|---|
| | 2-1 | 2-1 | 2-2 | 2-3 |
| Decamethylcyclopentasiloxane | 26. 0 | 20.0 | 18. 0 | 16. 0 |
| Dimethylpolysiloxane | 2.0 | 2.0 | 2.0 | 2.0 |
| Lauryl PEG-9 polydimethylsiloxyethyl dimethicone | 1.5 | 1.5 | 1.5 | 1.5 |
| Trimethylsiloxysilicic acid | 5.0 | 5.0 | 5.0 | 5.0 |
| Polyoxyethylene/methylpolysiloxane copolymer | 0.5 | 0.5 | 0.5 | 0.5 |
| Triglyceride isooctanoate | 5.0 | 5.0 | 5.0 | 5.0 |
| Cetyl isooctanoate | 5.0 | 5.0 | 5.0 | 5.0 |
| Isononyl isononanoate | 5.0 | 5.0 | 5.0 | 5.0 |
| 2-ethylhexyl paramethoxycinnamate | 7.5 | 7. 5 | 7.5 | 7.5 |
| Hexyl diethylaminohydroxy benzoylbenzoate | 2.0 | 2.0 | 2.0 | 2.0 |
| Benzotriazole derivative *1 | - | 1.0 | 3.0 | 5.0 |
| Dimethyldistearyl ammonium hectorite | 0.2 | 0.2 | 0.2 | 0.2 |
| Hydrophobicized titanium oxide | 12.0 | 12.0 | 12.0 | 12.0 |
| Purified water | 18.7 | 23.7 | 23.7 | 23.7 |
| Trisodium edetate | 0.05 | 0.05 | 0.05 | 0.05 |
| Dipropylene glycol | 5.0 | 5.0 | 5.0 | 5.0 |
| Paraben | 0.25 | 0.25 | 0.25 | 0.25 |
| Glycerin | 2.0 | 2.0 | 2.0 | 2.0 |
| Ethanol | 2.0 | 2.0 | 2.0 | 2.0 |
| Phenoxy ethanol | 0.3 | 0.3 | 0.3 | 0.3 |
| Total | 100.0 | 100.0 | 100.0 | 100.0 |

| | | | | |
|---|---|---|---|---|
| *1: 2-[4-(2-ethylhexyloxy)-2-hydroxyphenyl]-2H-benzotriazole | | | | |

### "Method for measuring the staining tendency"

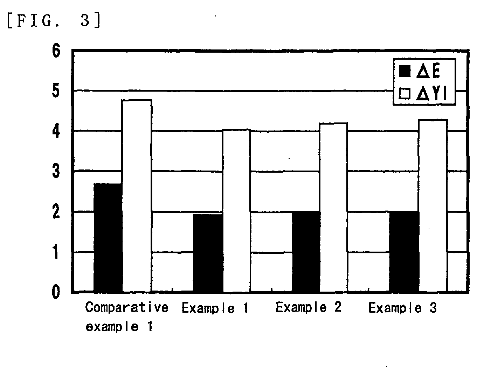

As shown in FIG. 1, the sample was applied thickly on an arm and then transferred to the middle of broad cotton (transferred amount approximately 0.06 g); after being left alone indoors for a day, it was washed using a conventional laundry detergent and Δ E and Δ YI were measured by using a spectrophotometer (CM-2002 from Minolta, currently Konica Minolta Sensing Co., Ltd.). The result is shown in Figure 3.

FIG. 3 indicates that the staining tendency of hexyl diethylaminohydroxybenzoylbenzoate decreases by the addition of the benzotriazole derivative. Even when the blend ratio is high, the staining tendency decreases compared with the case in which it is not added.

The following are formulation examples of the sunscreen cosmetic of the present invention. Each of these examples is a sunscreen cosmetic that has a reduced staining tendency on clothing and exhibits superior ultraviolet absorption capacity.

### "Example 2-4: Sunscreen cosmetic W/0 emulsion"

| | | wt% |
|---|---|---|
| 1. | Dimethylpolysiloxane | 1 |
| 2. | Decamethylcyclopentasiloxane | 25 |
| 3. | Trimethylsiloxysilicic acid | 5 |
| 4. | Polyoxyethylene-methylpolysiloxane copolymer | 1 |
| 5. | Lauryl PEG-9 polydimethylsiloxyethyl dimethicone | 1 |
| 6. | Isononyl isononoate | 5 |
| 7. | Dipropylene glycol | 5 |
| 8. | Dipotassium glycyrrhizate | 0.02 |
| 9. | Glutathione | 1 |
| 10. | Thiotaurine | 0.05 |
| 11. | Sophora flavescens extract | 1 |
| 12. | Paraben | Appropriate amount |
| 13. | Phenoxyethanol | Appropriate amount |
| 14. | 2-ethylhexyl paramethoxycinnamate | 7.5 |
| 15. | Dimethyldistearylammonium hectorite | 0.5 |
| 16. | Spherical polyalkyl acrylate powder | 5 |
| 17. | Butylethylpropanediol | 0.5 |
| 18. | Hexyl diethylaminohydroxybenzoylbenzoate | 2 |
| 19. | Benzotriazole derivative of Synthesis example 2 | 5 |
| 20. | Hydrophobicized zinc oxide or hydrophobicized titanium oxide | 15 |
| 21. | Purified water | Balance |

### Preparation method

The water phase was gradually added to the oil phase; after the addition a stirrer was used to homogenize the emulsified particles to complete the preparation.

### "Example 2-5: Sunscreen cosmetic O/W emulsion"

| | | wt% |
|---|---|---|
| 1. | Polyoxyethylene hydrogenated castor oil | 1 |
| 2. | Dimethicone copolyol | 0.5 |
| 3. | Decamethylcyclopentasiloxane | 15 |
| 4. | Isostearic acid | 0.5 |
| 5. | Phenyl trimethicone | 1 |
| 6. | Hydrophobicized titanium oxide | 5 |
| 7. | Hexyl diethylaminohydroxybenzoylbenzoate | 3 |
| 8. | 2-ethylhexyl-paramethoxycinnamate | 5 |
| 9. | Benzotriazole derivative of Synthesis example 1 | 2 |
| 10. | Silica | 1 |
| 11. | Citric acid | 0.01 |
| 12. | Sodium citrate | 0.09 |
| 13. | Paraben | Appropriate amount |
| 14. | Phenoxyethanol | Appropriate amount |
| 15. | Ethanol | 5 |
| 16. | Dynamite glycerin | 1 |
| 17. | Succinoglucan | 0.2 |
| 18. | Cellulose gum | 1 |
| 19. | Ion-exchanged water | Balance |

### Preparation method

After preparing the water phase, 10-19, it was gradually added to the oil phase, 1-9, followed by stirring by means of a homomixer.

### "Example 2-6: Sunscreen cosmetic O/W/O emulsion"

| | | wt% |
|---|---|---|
| 1. | Dimethylethyl polyoxyethylene (50) polyoxypropylene (40) block copolymer | 0.5 |
| 2. | Isostearic acid | 0.2 |
| 3. | 2-ethylhexyl paramethoxycinnamate | 7.5 |
| 4. | Benzotriazole derivative of Synthesis example 2 | 2.0 |
| 5. | Hexyl diethylaminohydroxybenzoylbenzoate | 2.0 |
| 6. | Decamethylcyclopentasiloxane | 35 |
| 7. | Polyoxybutylene polyoxypropylene glycol | 2.0 |
| 8. | Dimethyldistearylammonium hectorite | 0.5 |
| 9. | Hydrophobicized titanium oxide | 12.0 |
| 10. | Citric acid | 0.04 |
| 11. | Sodium citrate | 0.06 |
| 12. | Dipropylene glycol | 2.0 |
| 13. | Methyl glucose | 1. 0 |
| 14. | Dynamite glycerin | 1.0 |
| 15. | Sodium chloride | 0.1 |
| 16. | Methylparaben | Appropriate amount |
| 17. | Phenoxyethanol | Appropriate amount |
| 18. | Ion-exchanged water | Balance |

### Preparation method

The oil phase, 1-5, was gradually added to the water phase, 10-18, to obtain an 0/W preparation. This preparation was gradually added to the oil phase consisting of 6-9, followed by stirring using a homomixer to obtain the target emulsion.

### "Example 2-7: Self tanning cosmetic"

| | | wt% |
|---|---|---|
| Part A | | |
| 1. | 1,3-butylene glycol | 5 |
| 2. | Glycerin | 2 |
| 3. | Dihydroxyacetone | 5 |
| 4. | Disodium edetate | 0.05 |
| 5. | Paraben Appropriate | amount |
| 6. | Ion-exchanged water | Balance |

| Part B | | |
|---|---|---|
| 7. | Glyceryl stearate | 4 |
| 8. | Behenyl alcohol | 3 |
| 9. | Stearyl alcohol | 2 |
| 10. | Silicone oil | 1 |
| 11. | Hydrogenated palm oil | 2 |
| 12. | Liquid paraffin | 7 |
| 13. | Benzotriazole derivative of Synthesis example | 2 |
| 14. | Hexyl diethylaminohydroxybenzoylbenzoate | 2 |
| 15. | Perfume | Appropriate amount |

### Preparation method

Disodium edetate, dihydroacetone, glycerin, and Paraben heated and dissolved in 1,3-butylene glycol were added to the ion-exchanged water of part A. Each ingredient of part B was thoroughly dissolved and heated, and then added to part A, followed by emulsification. This was cooled to obtain the self tanning cream.

### "Example 2-8: Sunscreen cosmetic W/O emulsion"

| | | wt% |
|---|---|---|
| 1. | Dimethylpolysiloxane | 1 |
| 2. | Decamethylcyclopentasiloxane | 25 |
| 3. | Trimethylsiloxysilicic acid | 5 |
| 4. | Polyoxyethylene-methylpolysiloxane copolymer | 1 |
| 5. | Lauryl PEG-9 polydimethylsiloxyethyl dimethicone | 1 |
| 6. | Isononyl isononoate | 5 |
| 7. | Dipropylene glycol | 5 |
| 8. | Dipotassium glycyrrhizate | 0.02 |
| 9. | Glutathione | 1 |
| 10. | Thiotaurine | 0.05 |
| 11. | Sophora flavescens extract | 1 |
| 12. | Paraben | Appropriate amount |
| 13. | Phenoxyethanol | Appropriate amount |
| 14. | 2-ethylhexyl paramethoxycinnamate | 7.5 |
| 15. | Dimethyldistearylammonium hectorite | 0.5 |
| 16. | Spherical polyalkyl acrylate powder | 5 |
| 17. | Butylethylpropanediol | 0.5 |
| 18. | Hexyl diethylaminohydroxybenzoylbenzoate | 2 |
| 19. | Ethylhexyl 2-cyano-3,3-diphenylacrylate | 5 |
| 20. | Benzotriazole derivative of Synthesis example 2 | 5 |
| 21. | Hydrophobicized zinc oxide or hydrophobicized titanium oxide | 15 |
| 22. | Purified water | Balance |

### Preparation method

The water phase was gradually added to the oil phase; after the addition a stirrer was used to homogenize the emulsified particles to complete the preparation.

### "Example 2-9: Sunscreen cosmetic 0/W emulsion"

| | | wt% |
|---|---|---|
| 1. | Polyoxyethylene hydrogenated castor oil | 1 |
| 2. | Dimethicone copolyol | 0.5 |
| 3. | Decamethylcyclopentasiloxane | 15 |
| 4. | Isostearic acid | 0.5 |
| 5. | Phenyl trimethicone | 1 |
| 6. | Hydrophobicized titanium oxide | 5 |
| 7. | Hexyl diethylaminohydroxybenzoylbenzoate | 3 |
| 8. | Ethylhexyl 2-cyano-3,3-diphenylacrylate | 5 |
| 9. | 2-ethylhexyl-paramethoxycinnamate | 5 |
| 10. | Benzotriazole derivative of Synthesis example 1 | 2 |
| 11. | Silica | 1 |
| 12. | Citric acid | 0.01 |
| 13. | Sodium citrate | 0.09 |
| 14. | Paraben | Appropriate amount |
| 15. | Phenoxyethanol | Appropriate amount |
| 16. | Ethanol | 5 |
| 17. | Dynamite glycerin | 1 |
| 18. | Succinoglucan | 0.2 |
| 19. | Cellulose gum | 1 |
| 20. | Ion-exchanged water | Balance |

### Preparation method

The water phase, 11-20, was prepared, which was then gradually added to the oil phase, 1-10, followed by stirring by means of a homomixer.

### "Example 2-10: Sunscreen cosmetic O/W/O emulsion"

| | | wt% |
|---|---|---|
| 1. | Dimethylethyl polyoxyethylene (50) polyoxypropylene (40) block copolymer | 0.5 |
| 2. | Isostearic acid | 0.2 |
| 3. | 2-ethylhexyl paramethoxycinnamate | 7.5 |
| 4. | Benzotriazole derivative of Synthesis example 2 | 2.0 |
| 5. | Hexyl diethylaminohydroxybenzoylbenzoate | 2.0 |
| 6. | Ethylhexyl 2-cyano-3,3-diphenylacrylate | 5.0 |
| 7. | Decamethylcyclopentasiloxane | 35 |
| 8. | Polyoxybutylene polyoxypropylene glycol | 2.0 |
| 9. | Dimethyldistearylammonium hectorite | 0.5 |
| 10. | Hydrophobicized titanium oxide | 12.0 |
| 11. | Citric acid | 0.04 |
| 12. | Sodium citrate | 0.06 |
| 13. | Dipropylene glycol | 2.0 |
| 14. | Methyl glucose | 1.0 |
| 15. | Dynamite glycerin | 1.0 |
| 16. | Sodium chloride | 0.1 |
| 17. | Methylparaben | Appropriate amount |
| 18. | Phenoxyethanol | Appropriate amount |
| 19. | Ion-exchanged water | Balance |

### Preparation method

The oil phase, 1-6, was gradually added to the water phase, 11-19, to obtain an O/W preparation. This preparation was gradually added to the oil phase consisting of 7-10, followed by stirring using a homomixer to obtain the target emulsion.

### INDUSTRIAL APPLICABILITY

### [First invention]

The present invention can provide a sunscreen cosmetic that reduces the staining tendency of 4-(1,1-dimethylethyl)-4'-methoxydibenzoylmethane on clothing and exhibits superior ultraviolet absorption properties in the UV-A region and UV-B region.

### [Second invention]

The present invention can provide a sunscreen cosmetic that reduces the staining tendency of hexyl diethylaminohydroxybenzoylbenzoate on clothing and exhibits superior ultraviolet absorption properties in the UV-A region or UV-A-to-B region.

### [First and second inventions]

Since the staining tendency on clothing decreases, 4-(1,1-dimethylethyl)-4'-methoxydibenzoylmethane or hexyl diethylaminohydroxybenzoylbenzoate can be added at a high blend ratio and therefore SPF (Sun Protection Factor: UVB protection) and PF (Protection Grade of UVA: UVA protection) can be improved.

## Claims

1. A sunscreen cosmetic comprising 4-(1,1-dimethylethyl)-4'-methoxydibenzoylmethane, a benzotriazole derivative represented by the following general formula (I), and ethylhexyl 2-cyano-3,3-diphenylacrylate.
General formula (I) 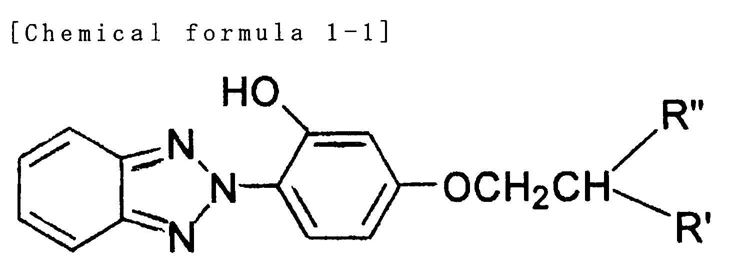 (In this formula, R' = a straight chain alkyl group of C1-C6 and R" = a straight chain alkyl group of C1-C3.)

2. The sunscreen cosmetic of claim 1 wherein said benzotriazole derivative is one, two or more chosen from a group consisting of 2-[4-(2-ethylhexyloxy)-2-hydroxyphenyl]-2H-benzotriazole and 2-(2-hydroxy-4-isobuthoxyphenyl)-2H-benzotriazole.

3. A sunscreen cosmetic comprising hexyl diethylaminohydroxybenzoylbenzoate and a benzotriazole derivative represented by the following general formula (I).
General formula (I) 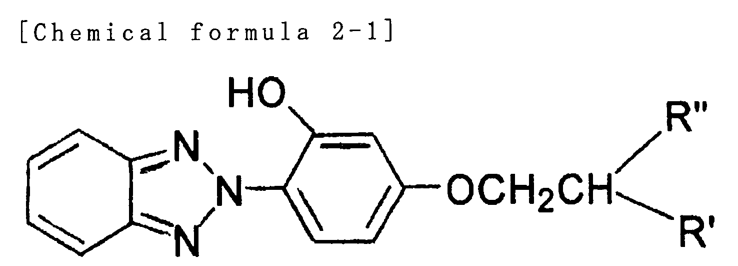 (In this formula, R' = a straight chain alkyl group of C1-C6 and R" = a straight chain alkyl group of C1-C3.)

4. The sunscreen cosmetic of claim 3 wherein said benzotriazole derivative is one, two or more chosen from a group consisting of 2-[4-(2-ethylhexyloxy)-2-hydroxyphenyl]-2H-benzotriazole and 2-(2-hydroxy-4-isobuthoxyphenyl)-2H-benzotriazole.

5. The sunscreen cosmetic of claim 3 or 4 additionally comprising ethylhexyl 2-cyano-3,3-diphenylacrylate.
